# EUROPEAN PATENT SPECIFICATION

(11) **EP 2 029 001 B1**
(45) Date of publication and mention of the grant of the patent: **12.11.2014**
(21) Application number: 07784091.6
(22) Date of filing: 24.05.2007
(51) Int. Cl.: A61B 5/00, A61B 6/00, A61B 5/11, G05B 23/02, A61B 6/03, G06F 19/00

(54) **COGNITIVE MONITORING WIRELESS DEVICE FOR HEALTHCARE EQUIPMENT**
DRAHTLOSE VORRICHTUNG ZUR KOGNITIVEN ÜBERWACHUNG FÜR EINE MEDIZINISCHE VORRICHTUNG
DISPOSITIF SANS FIL DE SURVEILLANCE COGNITIF POUR UN EQUIPEMENT DE SANTE

(30) Priority: 02.06.2006 US 803739 P
(43) Date of publication of application: 04.03.2009
(73) Proprietor: Koninklijke Philips N.V., 5656 AE Eindhoven (NL)
(72) Inventor: ALSAFADI, Yasser H., Yorktown Heights, New York 10598 (US)
(74) Representative: Verweij, Petronella Danielle
(86) International application number: PCT/US2007/069598
(87) International publication number: WO 2007/143402

(56) References cited:
- EP-A- 0 864 293
- WO-A-02/086835
- WO-A-2004/109992
- WO-A-2006/120600
- US-A1- 2005 021 373

## Description

The present application relates to wireless monitoring devices for healthcare equipment. It finds particular application for monitoring healthcare equipment, such as wireless medical sensors, diagnostic imaging systems, and the like. However, it is to be appreciated that this application is also applicable to monitoring other equipment including industrial equipment, manufacturing equipment, transportation equipment, residential and commercial building equipment, military equipment, and the like.

Healthcare equipment typically has numerous mechanical and electrical systems that can fail. Failure of these systems can render the equipment inoperative until it is repaired, leaving healthcare professionals unexpectedly without an important diagnostic or treatment tool. As one example, a CT scanner typically includes a rotating gantry which rotates on bearings which need maintenance, has power supplies for the x-ray tube, computer and other electronic equipment, cooling systems, a movable patient support, and the like, all of which may need maintenance or replacement. A CT scanner can have an automatic diagnostic system which monitors various conditions, such as arcing of the x-ray tube. When the arcing becomes too frequent, this information can be communicated to a central facility over a dedicated telephone line. Other conditions such as overheating bearings, processor malfunctions, and the like are similarly reportable.

All diagnostic equipment is not readily amenable to dedicated telephone lines. For example, a mobile ultrasound system can move from location to location. Transmitting monitored information from mobile equipment to a central station can be done in various ways, such as with a dedicated radio frequency signal. However, such mobile equipment may be operated in a variety of environments, such as a temperature controlled hospital in which thousands of electronic devices such as cell phones, PDAs, computers, healthcare monitors, and the like are all communicating wirelessly. Other times, the portable ultrasound system might be located in other environments, such as a field hospital in a dry hot desert, in a cool, damp mountain clinic, or the like.

Some healthcare equipment, such as patient worn physiological condition monitors, freely move among different sites in different environments and with different access to radio communications. Moreover, radio frequency bands are typically allocated on a national or regional basis. This requires such monitors to be manufactured specifically for each country. When the patient travels to another country, the communication frequency might be allocated for a different purpose, such as television or radio, which would strongly interfere with signals from the patient worn sensor.

Similar problems occur in other types of large stationary equipment, such as the machinery of a manufacturing facility, other types of mobile equipment such as automobiles, locomotives, and the like, and others.

EP0864293A1 describes a telemetry system comprising a transmitter for transmitting signals, preferably electromagnetic signals, and a receiver for receiving the signals from the transmitter. The receiver comprises a receiver contact unit for providing a contact with a transmitter contact unit of the transmitter. The receiver contact unit and the transmitter contact unit are adapted to provide a data communication during a contact phase for assigning a transmission channel to the transmitter and/or to the receiver. The receiver may further comprise means for monitoring a transmission activity in a pre-given channel range for determining possible channels in use, so that the transmission channel is assigned to the transmitter in accordance with the determined channels in use. The telemetry system is preferably used to for medical applications, such as pulse-oximetry or electrocardiography.

WO 2006/20600 A1 describes a patient monitoring system for monitoring the physiological state of a patient. The system includes a cognitive device for receiving data from sensors residing on the patient's body. Based on the received data the cognitive device decides what monitored information will be communicated to a remote location.

US 2005/0021373 A1 describes an apparatus for use in the medical field. The apparatus includes a communication device, which transmits data generated by a detector of said apparatus to a maintenance center in a wireless manner.

The present application proposes a cognitive monitoring wireless device according to claim 1 which overcomes the above-referenced problems and others.

In accordance with one aspect, a cognitive monitoring wireless apparatus is provided. A cognitive monitor is adapted to receive inputs from a plurality of sensors, generate messages indicative of at least some of the inputs received from the sensors, and decide, based on the sensed inputs, which messages should be forwarded. The apparatus further includes a cognitive radio which receives the messages from the cognitive monitor, selects among available communication parameters, and wirelessly communicates the messages.

In accordance with another aspect, a method of cognitive monitoring is provided according to claim 11. Inputs are received from a plurality of sensors. The messages indicative of at least some of the inputs received from the sensors are generated. Which of the messages should be forwarded is decided based on the sensed inputs. A selection among available communication parameters is made and the messages are wirelessly communicated.

One advantage is that it readily adapts to different types of healthcare and other equipment.

Another advantage is that it readily adapts to different environments.

Another advantage resides in reducing unpredicted downtime.

Still further advantages of the present invention will be appreciated to those of ordinary skill in the art upon reading and understand the following detailed description.

The invention may take form in various components and arrangements of components, and in various steps and arrangements of steps. The drawings are only for purposes of illustrating the preferred embodiments and are not to be construed as limiting the invention.
FIGURE 1 is a diagrammatic view of a cognitive monitoring system;
FIGURE 2 is a diagrammatic illustration of a decision support system for the cognitive monitor of FIGURE 1;
FIGURE 3 is a simplified implementation of a decision support system for the cognitive radio of FIGURE 1;
FIGURE 4 is a diagrammatic representation of components of the reasoner of FIGURES 2 and 3; and
FIGURE 5 illustrates an alternate embodiment in which the cognitive monitor is incorporated in a physiological condition monitor.

Machine and system condition monitoring is of vital importance to modern industry in its quest for high reliability, quality and efficiency. High costs in maintaining today's complex and sophisticated equipment create a need to enhance modern maintenance management systems. Condition-based maintenance (CBM) reduces the uncertainty of maintenance according to the needs indicated by the equipment condition. For example, online continuous monitoring enables the health of a plant to be continuously updated. Machinery can be shut down if a serious defect threatens an imminent catastrophic failure. Other, less urgent defects can be monitored and scheduled for repair at the next maintenance shutdown. Planning maintenance in advance enables spare parts to be ordered and all necessary manpower and resources to be available for the periodic maintenance, minimizing the downtime.

The growth of wireless services over the past several years demonstrates the vast and growing demand of businesses, customers, and governments for spectrum-based communication links. Spectrum access, efficiency, and reliability, are critical public policy issues. Advances in technology are creating the potential for radio systems to use more spectrum more intensely and with more efficiency than ever before. Among these advances are cognitive radio technologies that make possible more intensive and efficient spectrum uses by licensees within their own networks and by spectrum users sharing spectrum access on a negotiated or opportunistic basis. These technologies include, among other things, the ability of devices to determine their location, sense a spectrum in use by neighboring devices, change frequency, adjust power, and even alter transmission parameters and characteristics. These radio technologies open spectrum for use in space, time, and frequency dimensions that have previously been unavailable.

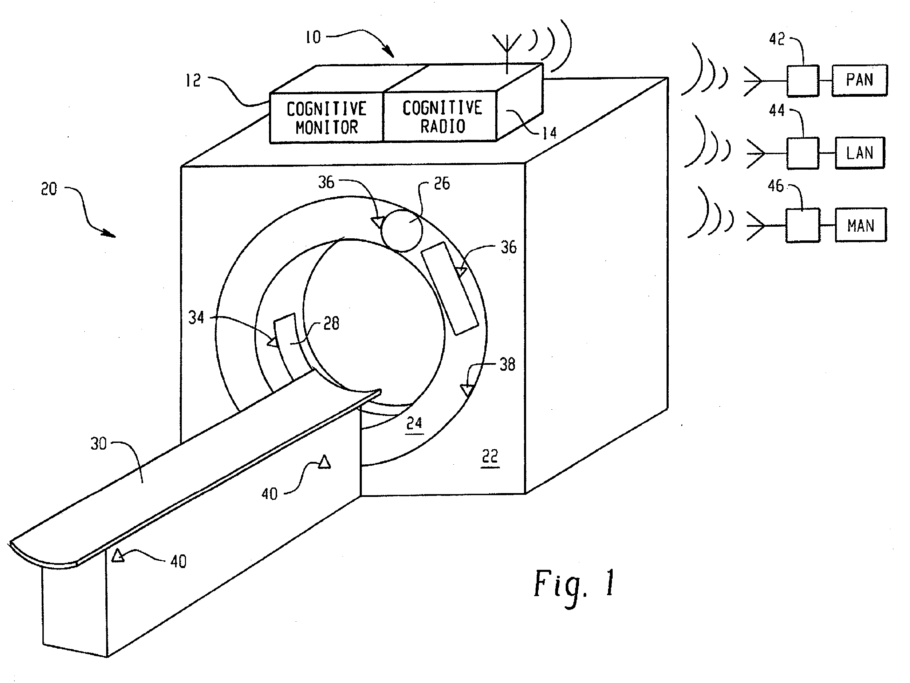

With reference to FIGURE 1, a cognitive device **10** gathers input from any of a variety of monitored devices, selects which monitored data should be communicated, and selects which mode of wireless communication to use. The cognitive device **10** includes a cognitive monitor **12** and a cognitive radio **14.** The cognitive monitor is an intelligent system that decides which monitored information should be communicated. This involves understanding the monitoring parameters, the equipment's condition, and the environment. The cognitive radio **14** is a radio that can change its transmitter parameters based on an interaction with the environment in which it operates. This interaction may involve active negotiation or communications with other spectrum users and/or passive sensing and decision making within the cognitive radio.

In the exemplary embodiment of FIGURE 1, the cognitive monitor is connected with a CT scanner **20.** The scanner includes a stationary gantry **22** and a rotating gantry **24.** An x-ray tube **26,** detectors **28,** and various other computers, cooling systems, coolant pumps, and the like are also mounted on the rotating gantry. The rotating gantry is supported on the stationary gantry by one or more bearings. A patient support **30** includes motors and gears for adjusting its height and for advancing the top into and out of the bore of the CT scanner. A plurality of sensors, such as a sensor **32** which senses arcing or other intermittent problems with the x-ray tube **26,** a monitor **34** for monitoring malfunctions of the x-ray detector, and other sensors **36** sense operation of the electronic equipment, the power supply, circulation of the coolant, coolant temperature, and the like. Additional sensors **38** on the stationary gantry monitor bearing temperature, the operation of electronic equipment on the stationary gantry, and the like. Additional monitors **40** on the patient support monitor operation of the motors and gears. The monitors **38** and **40** on the stationary gantry and the patient support communicate the sensed information to the cognitive monitor by wires. The sensors **32, 34, 36** on the rotating gantry can communicate their sensed information to the cognitive monitor via a slip ring, by RF communications, by optical communications, or the like. Of course, the CT scanner is illustrated by way of example only. Numerous other types of healthcare and non-healthcare equipment can also be outfitted with one or more sensors. Equipment is understood also to include small pieces of equipment, such as a patient-worn portable physiological condition monitor.

After collecting the data, the cognitive monitor **12** analyzes the ensemble of signals, using any of various techniques such as a logical alarm reduction algorithm (LARA) to identify erroneous or artifact signals versus true information signals. That is, the cognitive monitor sorts these signals according to their quality with artifacted signals being ranked low in quality versus artifact-free signals. The cognitive radio **14** selects the transmission protocol in accordance with the spectrum opportunities available. It may transmit to a receiver **42** of a personal area network (PAN), a receiver **44** of a local area network (LAN), a receiver **46** of a metropolitan area network (MAN), or the like.

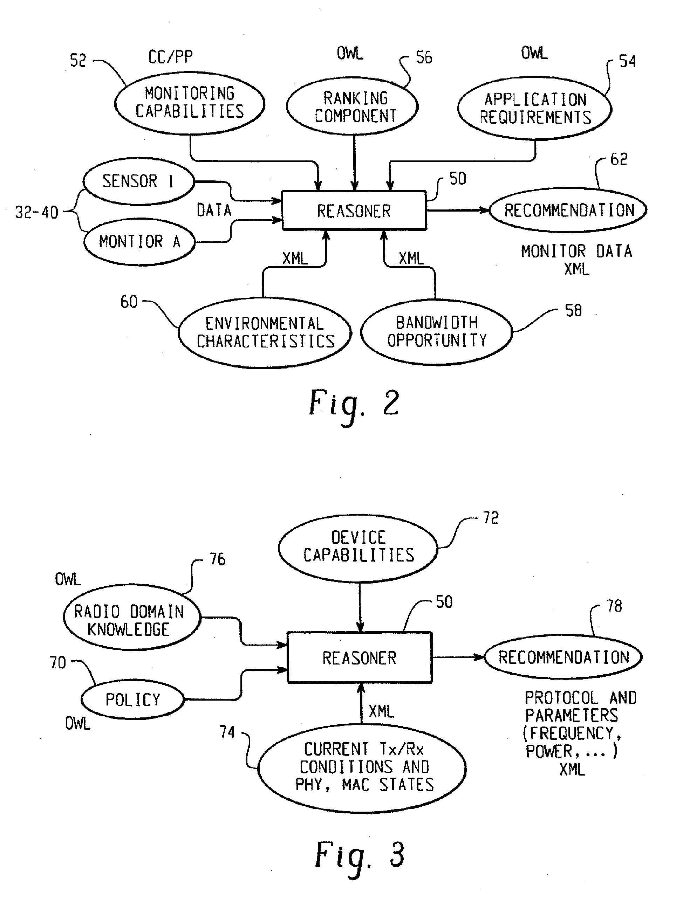

With reference to FIGURE 2, the cognitive monitor **12** includes a reasoner or logic processor **50.** The reasoner is connected with the plurality of sensors or monitors, such as sensors **32-40** discussed above, which input data collected from the monitored devices. The exact characteristics of the monitored and sensed data will vary in accordance with the sensors or monitors and may advantageously be described using composite capability/preference profile (CC/PP) structure and vocabulary in accordance with the W3C recommendation. With a generic cognitive monitor which is amenable to receiving different types of inputs, the monitoring capabilities **52** are input by an operator during initial set-up.

The reasoner further receives application requirements **54** describing the relationships among different monitored or sensed data. The requirements may include a set of rules regarding which data to communicate to the central location under which circumstances. For example, when sensing the temperature of the bearing, the application requirements will include a set of rules regarding how hot the bearing can get and at what temperatures information should be sent. For example, if the bearing is running 10% above normal, the rules may call for this information to be collected, summarized, and transmitted only daily or weekly. However, if the bearing temperature is escalating rapidly approaching a critical potential failure temperature, then the rules may call for the sensed temperatures to be communicated more frequently. The exact frequency may depend on the rate of temperature increase. When the bearing reaches the critical imminent failure temperature, the rules may call for that information to be sent immediately. Various other rules which describe the characteristics of the various sensed data which should be sent and the priority with which it should be sent are also contemplated. A ranking component **56** identifies erroneous signals or artifacts and helps differentiate the artifacts from artifact-free signals and ranks them accordingly. Artifact-free signals are ranked the highest and heavily artifacted signals are ranked the lowest. A bandwidth opportunity input **58** from the cognitive radio **14** advises the reasoner **50** of the amount of bandwidth available. The bandwidth opportunity information can, for example, be expressed in XML. Typically, the rules will specify that when there is very limited bandwidth available, only the highest priority information will be transmitted. By distinction, when a significant amount of bandwidth is available, lower priority information may be sent. In one embodiment, the various requirements are expressed in ontology web language (OWL).

The rules may specify different priorities when the equipment is operating under different environmental conditions. An environmental characteristics input **60** inputs information about the current usage environment. It captures appropriate information about location, time, temperature, humidity other environmental factors, the nature of the location such as home, office, factory, battle field, and the like. Information about rural versus city may also be input. Appropriate environmental condition sensors and inputs as may be appropriate to the significant environmental characteristics are connected with the one or more environmental characteristic inputs **60.** Inputs can be transmitted froma defined source or can be localized with the monitoring apparatus.

The reasoner **50** utilizes the ranking provided by the ranking component, the available bandwidth provided by the cognitive radio, the application requirements and other inputs in order to apply the rules in a logical analysis to generate a recommendation **62** regarding which input data or messages are to be transmitted. The rules utilized by the reasoner can be dynamic based on information or parameters from other sources.

The cognitive radio **14** selects the bandwidth opportunity which the cognitive monitor can use. With reference to FIGURE 3, an exemplary decision support system for the cognitive radio includes a policy description input **70** which describes constraints on transmission parameters to limit the level of interference received by primary radio systems in the area close to the secondary radio system. In the United States, the policy is prescribed by the Federal Communications Commission (FCC) and is represented in the OWL language using ontologies. A device capabilities input **72** receives a description of the characteristics and limitations of the device, such as its source of electrical power, CPU, memory, frequency range, channelization, modulation encoding scheme, and communication protocols. This can be described using the CC/PP recommendation.

A current transmit/receive conditions input **74** provides feedback about the condition of the current transmission environment, e.g., noisy, low chatter, etc. Measurement results are defined by various accepted standards, such as IEEE 802.11h, IEEE 802.11k, or the like, and may be described as ontology using the OWL language. A radio domain knowledge input **76** links to a repository of knowledge about the domain of radio communications. This includes such information as may be required about transmission parameters by algorithms for spectrum opportunity management. For example, transmission power, frequency, maximum distances between communicating devices, the modulation encoding schemes, and the like are related to each other. For example, the reasoner **50** may want to know if the device increases the transmission power, the detection range increases, and at the same time the level of interference that other radio devices would observe increases. There are many more interdependencies among the different radio transmission parameters. This base of information facilitates the generation of appropriate rules for the information, environment, the needed radio transmission distances, and the like which may be available.

Using the rules and these parameters, the reasoner **50** makes a logical decision and generates a further recommendation **78** that describes the parameters for transmission such as the frequency, the maximum allowed power, code, a particular protocol, and the like. The recommendation may, for example, be represented as an XML document or string.

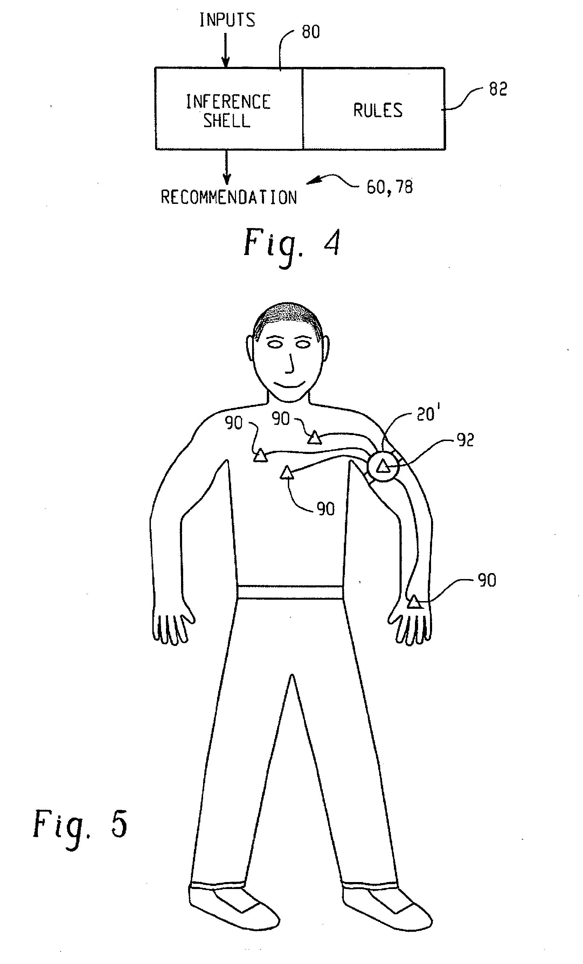

With reference to FIGURE 4, the reasoner component **50** can be implemented as an inference engine to derive the parameter recommendations based on inferences from previous inputs. The reasoner can identify several spectrum opportunities based on information from the policies, the current transmit/receive condition feedback, and device capabilities.

The cognitive radio is preferably a spectrum agile device. The rules utilized by the reasoner **50** are representations of the algorithms employed by such a spectrum agile device. These can be modeled using Protégé, for example. A rule engine, such as JESS, in a Java environment is used as an inference engine or shell **80.**

The described device provides a platform which the user can tailor or program to any of a multitude of equipment types by appropriate loading the rules and other inputs into a rules memory **82.** The rules are dynamic, rather than static. That is, the rules are updated and changed as sensors, industry and government standards, technology, and the like change or are updated. These devices can be deployed anywhere in the world including in different markets with different available spectrums and will still be able to communicate alarms. Custom tailored mobile security or environmental monitoring systems can be created with intelligent alarms that are always in communication with authorities. Such a device reports useful information about the condition of any subsystem, system, major component, and the like, any time from any place by detecting the spectrum opportunities and establishing a wireless communication link.

With reference to FIGURE 5, the cognitive monitor **12** is housed in a physiological condition monitor **20'.** Sensors **90,** such as EKG, pulse rate, blood oxygen, and other sensors input sensed information into the portable monitor **20'** and the cognitive device **10.** Additional sensors **92** for sensing battery levels and potential malfunctions or maintenance indicators in the monitor **20'** are connected with the cognitive monitor **12** to input signals indicative of the sensed conditions. As described above, the cognitive monitor decides, based on various rules and inputs, which of the received sensor inputs should be transmitted to a central location and with what priority.

## Claims

1. A cognitive monitoring wireless apparatus **(10, 10')** comprising:
a cognitive monitor **(12)** which is adapted to receive inputs from a plurality of sensors, generate messages indicative of at least some of the inputs received from the sensors, and decide based on the sensed inputs which of the messages should be forwarded;
a cognitive radio **(14)** which is adapted to receive the forwarded messages from the cognitive monitor (12), select among available communication parameters, and wirelessly communicate the messages, wherein
the plurality of sensors includes sensors **(32-40, 92)** connected with a healthcare apparatus **(20, 20')** for sensing operating conditions of the healthcare apparatus (20, 20').

2. The apparatus according to claim 1, wherein the cognitive monitor (14) includes:
a reasoner **(50)** which is adapted to perform a logical analysis of the input from the sensors based on rules and other available information.

3. The apparatus according to claim 2, wherein the cognitive monitor **(12)** further includes at least one of:
a monitoring capabilities input **(52)** which is adapted to receive characteristics of the input from the sensors and the sensors for use in the logic analysis;
an application requirements input **(54)** which is adapted to receive a description of relationships among different potential inputs for use in the logical analysis;
a ranking component input which is adapted to provide data for determining a degree of artifacting of the inputs received from the sensors and rank the inputs accordingly;
a bandwidth opportunity input **(58)** which is adapted to receive characteristics of available bandwidth from the cognitive radio **(14)** for use in the logical analysis; and,
an environmental characteristics input **(60)** which is adapted to receive data describing a current usage environment for use in the logical analysis.

4. The apparatus according to claim 3, wherein the logical analysis performed by the reasoner **(50)** uses the ranking provided by the ranking component input **(56),** the available bandwidth provided by the bandwidth opportunity input **(58)** from the cognitive radio, the application requirements provided by the application requirements input **(58),** and other inputs to determine which messages should be transmitted.

5. The apparatus according to claim 2, wherein the reasoner **(50)** is adapted to make logical decisions concerning transmission properties of the cognitive radio **(14),** the reasoner further including at least one of:
a policy description input **(70)** which is adapted to receive regulatory constraints on transmission parameters for use in making the logical decisions;
a device capabilities input **(72)** which is adapted to receive input describing characteristics and limitations of the cognitive radio for use in the logical decision making;
a current transmit/receive conditions input **(74)** which is adapted to receive data describing a current transmission environment experienced by the cognitive radio;
a radio domain knowledge input **(76)** which is adapted to receive algorithms for spectrum opportunity management.

6. The apparatus according to claim 5, wherein the logical decision making performed by the reasoner **(50)** generates a recommendation **(78)** which describes the radio frequency transmission parameters to be used by the cognitive radio **(14).**

7. The apparatus according to claim 6, wherein the transmission parameters include frequency, maximum allowed power, code, and protocol.

8. The apparatus according to claim 1, wherein the healthcare device is one of an imager **(20)** and a portable patient carried physiological condition monitor **(20').**

9. The apparatus according to claim 1, further including a plurality of sensors **(90)** which are adapted to be affixed to a patient to measure physiological conditions, the sensors being connected with the cognitive monitor **(12).**

10. The apparatus according to claim 1, wherein the cognitive monitor **(12)** includes an inference engine **(80)** and a memory **(82)** which is adapted to store a plurality of rules received from rules inputs, the inference engine is adapted to analyze the received inputs from the sensors in accordance with the rules to decide which information should be sent to a remote monitoring station.

11. A cognitive monitoring method comprising:
receiving inputs from a plurality of sensors;
generating one or more messages indicative of at least some of the inputs received from the sensors, deciding based on the sensed inputs which of the messages should be forwarded;
selecting among available communication parameters; and,
wirelessly communicating the messages to be forwarded, and
sensing operating conditions of a healthcare (20, 20') apparatus with the sensors.

12. The method according to claim 11, wherein selecting among available communication parameters includes selecting among frequency, maximum allowed power, code, and protocol.

13. The method as set forth in claim 11, further including:
performing a logical analysis on the inputs from the sensors based on at least one of:
monitoring capabilities which describe characteristics of the input from the sensors and the sensors;
application requirements which describe relationships among different potential inputs;
ranking data from which a degree of artifacts in the received inputs from the sensors can be determined and the inputs ranked;
bandwidth opportunity which describes characteristics of available bandwidth for wirelessly communicating the messages; and,
environmental characteristics which describe a current usage environment.

14. The method according to claim 11, further including:
making logical decisions concerning communication parameters based on at least one of:
policy description which describes regulatory constraints on transmission parameters;
device capabilities describing characteristics and limitations of a cognitive radio which selects the communication format and wirelessly communicates the messages;
current transmit/receive conditions which describe a current transmission environment experienced by the cognitive radio;
radio domain knowledge which includes algorithms for spectrum opportunity management.

## Patentansprüche

1. Drahtloses Gerät zur kognitiven Überwachung (10, 10'), das Folgendes umfasst:
einen kognitiven Monitor (12), der dafür ausgelegt ist, Eingangssignale von einer Vielzahl von Sensoren zu empfangen, Meldungen zu erzeugen, die auf mindestens einige der von den Sensoren empfangenen Eingangssignale hindeuten, und basierend auf den erfassten Eingangssignalen zu entscheiden, welche der Meldungen weiterzuleiten sind;
eine kognitive Funkeinheit (14), die dafür ausgelegt ist, die weitergeleiteten Meldungen von dem kognitiven Monitor (12) zu empfangen, unter verfügbaren Kommunikationsparametern auszuwählen und die Meldungen drahtlos zu übertragen, wobei
die Vielzahl von Sensoren Sensoren (32-40, 92) umfasst, die mit einem medizinischen Gerät (20, 20') verbunden sind, um die Betriebsbedingungen des medizinischen Geräts (20, 20') zu erfassen.

2. Gerät nach Anspruch 1, wobei der kognitive Monitor (14) Folgendes umfasst:
einen Reasoner (50), der dafür ausgelegt ist, basierend auf Regeln und anderen verfügbaren Informationen eine Logikanalyse der von den Sensoren stammenden Eingangssignale durchzuführen.

3. Gerät nach Anspruch 2, wobei der kognitive Monitor (12) weiterhin mindestens eines von folgenden Elementen umfasst:
einen Eingang für Überwachungsfähigkeiten (52), der dafür ausgelegt ist, Eigenschaften des Eingangssignals von den Sensoren und den Sensoren zur Verwendung in der Logikanalyse zu empfangen;
einen Eingang für die Anwendungsanforderungen (54), der dafür ausgelegt ist, eine Beschreibung der Beziehungen unter verschiedenen möglichen Eingängen zur Verwendung in der Logikanalyse zu empfangen;
einen Eingang für die Komponenten-Rangeinstufung, der dafür ausgelegt ist, Daten zum Ermitteln eines Grad der Artefaktbeeinflussung der von den Sensoren empfangenen Eingangssignale zu ermitteln und die Eingangssignale entsprechend einzustufen;
einen Eingang für die Bandbreitenmöglichkeit (58), der dafür ausgelegt ist, Eigenschaften der verfügbaren Bandbreite von der kognitiven Funkeinheit (14) zur Verwendung in der Logikanalyse zu empfangen; und
einen Eingang für Umgebungseigenschaften (60), der dafür ausgelegt ist, Daten, welche eine aktuelle Nutzungsumgebung beschreiben, zur Verwendung in der Logikanalyse zu empfangen.

4. Gerät nach Anspruch 3, wobei für die durch den Reasoner (50) durchgeführte Logikanalyse die von dem Eingang für die Komponenten-Rangeinstufung (56) bereitgestellte Rangeinstufung, die von dem Eingang für die Bandbreitenmöglichkeit (58) bereitgestellte verfügbare Bandbreite von der kognitiven Funkeinheit, die von dem Eingang für die Anwendungsanforderungen (54) bereitgestellten Anwendungsanforderungen und andere Eingangssignale verwendet werden, um zu ermitteln, welche Meldungen übertragen werden sollten.

5. Gerät nach Anspruch 2, wobei der Reasoner (50) dafür ausgelegt ist, logische Entscheidungen bezüglich der Übertragungseigenschaften des kognitiven Funkeinheit (14) zu treffen, wobei der Reasoner weiterhin mindestens eines der folgenden Elemente umfasst:
einen Eingang für die Politikbeschreibung (70), der dafür ausgelegt ist, regulatorische Einschränkungen für Übertragungsparameter zur Verwendung beim Treffen der logischen Entscheidungen zu empfangen;
einen Eingang für Gerätefähigkeiten (72), der dafür ausgelegt ist, den Eingangssignale, welche die Eigenschaften und Begrenzungen der kognitiven Funkeinheit beschreiben, zur Verwendung beim Treffen der zu logischen Entscheidung zu empfangen;
einen Eingang für die aktuellen Sende/Empfangsbedingungen (74), der dafür ausgelegt ist, Daten zu empfangen, die eine aktuelle Übertragungsumgebung beschreiben, welche durch die kognitive Funkeinheit erfahren wird;
einen Eingang für Funkbereichkenntnisse (76), der dafür ausgelegt ist, Algorithmen für das Spektrummöglichkeitensmanagement zu empfangen.

6. Gerät nach Anspruch 5, wobei die durch den Reasoner (50) durchgeführte logische Entscheidungsfindung eine Empfehlung (78) hervorbringt, die die durch die kognitive Funkeinheit (14) zu verwendenden Funkfrequenzübertragungsparameter beschreibt.

7. Gerät nach Anspruch 6, wobei die Übertragungsparameter Frequenz, maximal zulässige Leistung, Code und Protokoll umfassen.

8. Gerät nach Anspruch 1, wobei die medizinische Vorrichtung entweder ein Bildgebungssystem (20) oder ein von dem Patienten getragener portabler Monitor zur Überwachung des physiologischen Zustands (20') ist.

9. Gerät nach Anspruch 1, weiterhin umfassend eine Vielzahl von Sensoren (90), die dafür ausgelegt sind, an einem Patienten angebracht zu werden, um physiologische Zustände zu messen, wobei die Sensoren mit dem kognitiven Monitor (12) verbunden sind.

10. Gerät nach Anspruch 1, wobei der kognitive Monitor (12) eine Inferenzmaschine (80) und einen Speicher (82) umfasst, der dafür ausgelegt ist, eine Vielzahl von Regeln, die von den Regeleingängen empfangen wurden, zu speichern, wobei die Inferenzmaschine dafür ausgelegt ist, die empfangenen Eingangssignale von den Sensoren in Übereinstimmung mit den Regeln zu analysieren, um zu entscheiden, welche Informationen an eine abgesetzte Überwachungsstation gesendet werden sollten.

11. Kognitives Überwachungsverfahren, das Folgendes umfasst:
Empfangen von Eingangssignalen von einer Vielzahl von Sensoren;
Erzeugen von einer oder mehreren Meldungen, die auf mindestens einige der von den Sensoren empfangenen Eingangssignale hindeuten, und basierend auf den erfassten Eingangssignalen entscheiden, welche der Meldungen weiterzuleiten sind;
Auswählen unter verfügbaren Kommunikationsparametern; und
Erfassen der Betriebsbedingungen eines medizinischen Geräts (20, 20') mit den Sensoren.

12. Verfahren nach Anspruch 11, wobei das Auswählen unter verfügbaren Kommunikationsparametern das Auswählen unter Frequenz, maximal zulässiger Leistung, Code und Protokoll umfasst.

13. Verfahren nach Anspruch 11, das weiterhin Folgendes umfasst:
Durchführen einer Logikanalyse an den Eingangssignalen von den Sensoren basierend auf mindestens einem der folgenden Elemente:
Überwachungsfähigkeiten, die Eigenschaften des Eingangssignals von den Sensoren und die Sensoren beschreiben;
Anwendungsanforderungen, die die Beziehungen unter verschiedenen möglichen Eingängen beschreiben;
Rangeinstufungsdaten, anhand von denen ein Grad der Artefaktbeeinflussung der von den Sensoren empfangenen Eingangssignale ermittelt werden kann und die Eingangssignale entsprechend eingestuft werden können;
Bandbreitenmöglichkeit, welche die Eigenschaften der verfügbaren Bandbreite für das drahtlose Kommunizieren der Meldungen beschreibt; und
Umgebungseigenschaften, die eine aktuelle Nutzungsumgebung beschreiben.

14. Verfahren nach Anspruch 11, das weiterhin Folgendes umfasst:
Treffen von logischen Entscheidungen bezüglich der Kommunikationsparameter basierend auf mindestens einem der folgenden Elemente:
Politikbeschreibung, die regulatorische Einschränkungen für Übertragungsparameter beschreibt;
Gerätefähigkeiten, die Eigenschaften und Begrenzungen einer kognitiven Funkeinheit beschreiben, welche das Kommunikationsformat auswählt und die Meldungen drahtlos kommuniziert;
aktuelle Sende/Empfangsbedingungen, die eine aktuelle Übertragungsumgebung beschreiben, die durch die kognitive Funkeinheit erfahren wird;
Funkbereichskenntnisse, die Algorithmen für das Spektrummöglichkeitsmanagement umfassen.

## Revendications

1. Appareil sans fil de surveillance cognitif (10, 10') comprenant :
un moniteur cognitif (12) qui est conçu pour recevoir des entrées en provenance d'une pluralité de capteurs, générer des messages indicatifs d'au moins certaines des entrées reçues des capteurs, et décider sur la base des entrées détectées lesquels des messages doivent être transférés ;
une radio cognitive (14) qui est conçue pour recevoir les messages transférés en provenance du moniteur cognitif (12), effectuer une sélection parmi les paramètres de communication disponibles et communiquer sans fil les messages, dans lequel
la pluralité de capteurs inclut les capteurs (32-40, 92) connectés à un appareil de santé (20, 20') pour détecter les conditions opérationnelles de l'appareil de santé (20, 20').

2. Appareil selon la revendication 1, dans lequel le moniteur cognitif (14) inclut :
un raisonneur (50) qui est conçu pour effectuer une analyse logique de l'entrée des capteurs sur la base des règles et autres informations disponibles.

3. Appareil selon la revendication 2, dans lequel le moniteur cognitif (12) inclut en outre au moins une parmi :
une entrée de capacités de surveillance (52) qui est conçue pour recevoir des caractéristiques de l'entrée en provenance des capteurs et des capteurs destinés à être utilisés dans l'analyse logique ;
une entrée des exigences d'application (54) qui est conçue pour recevoir une description de relations parmi différentes entrées potentielles destinées à être utilisées dans l'analyse logique ;
une entrée de composante de classement qui est conçue pour fournir des données pour déterminer un degré d'artefact des entrées reçues en provenance des capteurs et classer les entrées en conséquence ;
une entrée d'opportunité de largeur de bande (58) qui est conçue pour recevoir les caractéristiques de largeur de bande disponible en provenance de la radio cognitive (14) destinées à être utilisées dans l'analyse logique ; et
une entrée de caractéristiques environnementales (60) qui est conçue pour recevoir des données décrivant un environnement d'usage courant destinés à être utilisées dans l'analyse logique.

4. Appareil selon la revendication 3, dans lequel l'analyse logique réalisée par le raisonneur (50) utilise le classement fourni par l'entrée de composante de classement (56), la largeur de bande disponible fournie par l'entrée d'opportunité de largeur de bande (58) en provenance de la radio cognitive, les exigences d'application fournies par l'entrée d'exigences d'application (54), et d'autres entrées pour déterminer quels messages doivent être transmis.

5. Appareil selon la revendication 2, dans lequel le raisonneur (50) est conçu pour prendre des décisions logiques concernant des propriétés de transmission de la radio cognitive (14), le raisonneur incluant en outre au moins une parmi :
une entrée de description de politique (70) qui est conçue pour recevoir des contraintes réglementaires sur les paramètres de transmission destinés à être utilisés dans la prise de décisions logiques ;
une entrée de capacités de dispositif (72) qui est conçue pour recevoir l'entrée décrivant des caractéristiques et des limites de la radio cognitive destinées à être utilisées dans la prise de décision logique ;
une entrée de conditions de transmission/réception actuelle (74) qui est conçue pour recevoir des données décrivant un environnement de transmission courant expérimenté par la radio cognitive ;
une entrée de connaissance de domaine radio (76) qui est conçue pour recevoir des algorithmes de gestion d'opportunités de spectre.

6. Appareil selon la revendication 5, dans lequel la prise de décision logique réalisée par le raisonneur (50) génère une recommandation (78) qui décrit les paramètres de transmission radiofréquence devant être utilisés par la radio cognitive (14).

7. Appareil selon la revendication 6, dans lequel les paramètres de transmission incluent la fréquence, la puissance autorisée maximale, le code et le protocole.

8. Appareil selon la revendication 1, dans lequel le dispositif de santé est l'un d'un imageur (20) et d'un moniteur de condition physiologique portable porté par un patient (20').

9. Appareil selon la revendication 1, incluant en outre une pluralité de capteurs (90) qui sont conçus pour être fixés à un patient pour mesurer les conditions physiologiques, les capteurs étant connectés avec le moniteur cognitif (12).

10. Appareil selon la revendication 1, dans lequel le moniteur cognitif (12) inclut un moteur d'inférence (80) et une mémoire (82) qui est conçue pour stocker une pluralité de règles reçues d'entrées de règles, le moteur d'inférence étant conçu pour analyser les entrées reçues en provenance des capteurs en fonction des règles pour décider quelles informations doivent être envoyées à une station de surveillance distante.

11. Procédé de surveillance cognitif comprenant :
la réception d'entrées en provenance d'une pluralité de capteurs ;
la génération d'un ou plusieurs messages indicatifs d'au moins certaines des entrées reçues en provenance des capteurs, en décidant, sur la base des entrées détectées, lesquels des messages doivent être transférés;
la sélection parmi les paramètres de communication disponibles ; et,
la communication sans fil des messages devant être transférés, et
la détection de conditions opérationnelles d'un appareil de santé (20, 20') à l'aide des capteurs.

12. Procédé selon la revendication 11, dans lequel la sélection parmi les paramètres de communication disponibles inclut la sélection parmi la fréquence, la puissance autorisée maximale, le code et le protocole.

13. Procédé selon la revendication 11, incluant en outre :
la réalisation d'une analyse logique sur les entrées en provenance des capteurs sur la base d'au moins une parmi :
la surveillance des capacités qui décrivent les caractéristiques de l'entrée en provenance des capteurs et des capteurs ;
l'application des exigences qui décrivent les relations entre différentes entrées potentielles ;
le classement des données à partir desquelles un degré d'artéfacts des entrées reçues des capteurs peut être déterminé et des entrées classées ;
l'opportunité de largeur de bande qui décrit les caractéristiques de la largeur de bande disponible pour la communication sans fil des messages ; et
les caractéristiques environnementales qui décrivent un environnement d'usage courant.

14. Procédé selon la revendication 11, incluant en outre :
la prise de décisions logiques concernant les paramètres de communication sur la base d'au moins une parmi :
la description de politique qui décrit les contraintes réglementaires sur les paramètres de transmission ;
les capacités du dispositif décrivant les caractéristiques et les limites d'une radio cognitive qui sélectionne le format de communication et communique les messages sans fil :
les conditions de transmission/réception courantes qui décrivent un environnement de transmission courant expérimenté par la radio cognitive ;
la connaissance du domaine radio qui inclut les algorithmes pour la gestion des opportunités de spectre.
